(19) Europäisches Patentamt
European Patent Office
Office européen des brevets

(11) **EP 2 653 153 B1**

(12) **EUROPEAN PATENT SPECIFICATION**

(45) Date of publication and mention
of the grant of the patent:
**27.05.2015 Bulletin 2015/22**

(51) Int Cl.:
*A61K 9/00* *(2006.01)*     *A61K 31/357* *(2006.01)*
*A61K 47/44* *(2006.01)*     *A61P 33/06* *(2006.01)*

(21) Application number: **13176933.3**

(22) Date of filing: **27.10.2008**

(54) **ANTI-MALARIAL PHARMACEUTICAL COMPOSITION**

PHARMAZEUTISCHES ANTI-MALARIA MITTEL

COMPOSITION PHARMACEUTIQUE ANTIPALUDIQUE

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **25.10.2007 GB 0720967
10.04.2008 GB 0806510**

(43) Date of publication of application:
**23.10.2013 Bulletin 2013/43**

(62) Document number(s) of the earlier application(s) in
accordance with Art. 76 EPC:
**08806806.9 / 2 209 464**

(73) Proprietor: **Malaria Research Company Pty. Ltd.
Osborne Park, WA 6017 (AU)**

(72) Inventors:
• **Booles, Clive**
  **Norwich, Norfolk NR4 7GJ (GB)**
• **ROSS, Calvin (Deceased)**
  **deceased (GB)**
• **SAMS, Martin**
  **Norwich, Norfolk NR4 GJ (GB)**

(74) Representative: **Harris, Oliver John Richard et al
Novagraaf UK
Centrum
Norwich Research Park
Colney Lane
Norwich NR4 7UG (GB)**

(56) References cited:
**WO-A-2007/043057      US-A1- 2003 203 875
US-A1- 2004 038 933**

• **TOUITOU ET AL: "Treatment of malaria in a
mouse model by intranasal drug administration",
INTERNATIONAL JOURNAL OF
PARASITOLOGY, PERGAMON PRESS, GB, vol.
36, no. 14, 19 November 2006 (2006-11-19), pages
1493-1498, XP005774092, ISSN: 0020-7519**
• **NAVARATNAM V ET AL: "PHARMACOKINETICS
OF ARTEMISININ-TYPE COMPOUNDS",
CLINICAL PHARMACOKINETICS, ADIS
INTERNATIONAL LTD., AUCKLAND, NZ, vol. 39,
no. 4, 1 October 2000 (2000-10-01), pages 255-270,
XP009012253, ISSN: 0312-5963**

## Description

<u>Field of the Invention</u>

The invention relates to pharmaceutical compositions for use in methods for the treatment of uncomplicated and complicated malaria.

<u>Background and Prior Art Known to the Applicant</u>

Malaria is an infectious disease widespread in many tropical and subtropical regions, caused by the infectious parasite *Plasmodium* transmitted primarily by the female mosquito of the *Anopheles* genus. Malaria is the cause of between one and three million deaths annually, mostly in sub-Saharan Africa. Of these, some 75% are of children under five.

Many pharmaceuticals have been developed or trialled for the treatment or prevention of malaria in both children and adults. Although useful pharmaceutical agents exist, and the life cycle of the malaria-carrying mosquito is well understood, practical intervention strategies have so far failed to bring this disease under control. As with most infectious diseases, issues of drug resistance are ever-present. However, for malaria, other confounding factors include the difficulty of administration of drugs to those in need, especially to children. In the most severely affected regions, children are often undernourished and suffer from other ailments. Apart from the symptoms caused by the malaria infection itself, episodes of diarrhoea and vomiting are not uncommon in such children. As a result, children are unable to swallow medicines in tablet form and it is extremely difficult to find appropriate veins in children for administration by the intravenous route. Even if this were possible, in many cases there are no trained medical personnel on hand to administer drugs intravenously, especially where a course of medication is required over a period of days or weeks.

Amongst the active pharmaceuticals of use in the treatment of malaria are a number of compounds derived from artemesenin, a sesquiterpene lactone endoperoxide originally isolated from *Artemesia annua* (Woodrow et al. Postgrad. Med.J. 2005; 81:71-78). These compounds include the semi-synthetic derivatives artenimol, artesunate, artemether and arteether (artemotil). The International Pharmacopoeia (*Ph. Int.,* World Health Organisation) lists a number of these for the treatment of malaria, *viz:* Artemether in the form of capsules, tablets or an injectable formulation; Artemesenin in the form of capsules or tablets; arteether in an injectable formulation; and both artenimol and artesunate in the form of tablets.

US Patent 6306896 describes pharmaceutically active compositions containing artemisinine and/or derivatives of artemisinine. The active ingredients are formulated for rectal administration, in the form of suppositories. Rectal administration of antimalarial therapeutics is particularly problematic, for a number of reasons: Firstly, many people suffering with malaria experience diarrhoea, making administration difficult. Secondly, for effective absorption through the rectal mucosa, patients need to have good nutritional status and a good diet, containing a high fat content; this is rarely the case in sub-Saharan Africa. Thirdly, in many communities affected by malaria, there are strong cultural barriers to the use and administration of suppositories.

It can be seen that all of these formulations face the difficulties of administration described above. It is therefore amongst the objects of the present invention to address these and other issues.

<u>Summary of the Invention</u>

Accordingly, the invention provides a pharmaceutical composition for use in a method of preventing or treating malaria, said composition comprising: artemether or arteether; and a pharmaceutically-acceptable excipient selected from the group consisting of: medium chain length triglycerides; short chain triglycerides; omega-3-marine triglycerides and fish oil, rich in omega-3-acids, said composition formulated for transmucosal sublingual, buccal or nasal dosage.

The inventors have found that the transmucosal sub-lingual, transmucosal buccal and transmucosal nasal routes for administration of artemether or arteether are effective for delivery of the pharmaceutical into the systemic circulation e.g. for the treatment of malaria. Furthermore, for the first time, it provides an administration route that is acceptable to children requiring treatment, and that may be administered by non-medically qualified personnel. It has particular advantage, therefore, in more remote village settings, where e.g. village elders can be trained in the diagnosis of malaria, and subsequent administration of the drug. The composition can be delivered e.g. sublingually as a liquid bolus, or, more preferably, as a spray.

Medium chain length triglycerides are defined in the European Pharmacopoeia Monograph 0868, as:

A mixture of triglycerides of saturated fatty acids, mainly of caprylic acid (octanoic acid, $C_8H_{16}O_2$) and of capric acid (decanoic acid, $C_{10}H_{20}O_2$). Medium-chain triglycerides are obtained from the oil extracted from the hard, dried fraction of the endosperm of *Cocos nucifera* L. or from the dried endosperm of *Elaeis guineensis* Jacq. When Medium-chain Triglycerides are prepared from the endosperm of *Cocos nucifera* L., the title Fractionated Coconut Oil may be used. Medium chain length triglycerides have a minimum 95.0 per cent of saturated fatty acids with 8 and 10 carbon atoms. Further chemical and physical properties are described in the European Pharmacopoeia Monograph 0868, and equivalent documents.

Short chain triglycerides are triglycerides having chain lengths of less than 6 carbon atoms.

Omega-3-marine triglycerides are defined in the European Pharmacopoeia Monograph 0868 as mixture of mono-, di- and triesters of omega-3 acids with glycerol containing mainly triesters and obtained either by esterification of concentrated and purified omega-3 acids with glycerol or by transesterification of the omega-3 acid ethyl esters with glycerol. The origin of the omega-3 acids is the body oil from fatty fish species coming from families like *Engraulidae, Carangidae, Clupeidae, Osmeridae, Salmonidae* and *Scombridae.* The omega-3 acids are identified as the following acids: alpha-linolenic acid (C18:3 n-3), moroctic acid (C18:4 n-3), eicosatetraenoic acid (C20:4 n-3), timnodonic (eicosapentaenoic) acid (C20:5 n-3; EPA), heneicosapentaenoic acid (C21:5 n-3), clupanodonic acid (C22:5 n-3) and cervonic (docosahexaenoic) acid (C22:6 n-3; DHA). The sum of the contents of the omega-3 acids EPA and DHA, expressed as triglycerides is a minimum of 45.0 per cent, and the total omega-3 acids, expressed as triglycerides is a minimum of 60.0 per cent. Tocopherol may be added as an antioxidant.

Fish oil, rich in omega-3-acids is also defined in the European Pharmacopeia as purified, winterised and deodorised fatty oil obtained from fish of the families *Engraulidae, Carangidae, Clupeidae, Osmeridae, Scombridae* and *Ammodytidae.* The omega-3 acids are defined as the following acids: *alpha*-linolenic acid (C18:3 n-3), moroctic acid (C18:4 n-3), eicosatetraenoic acid (C20:4 n-3), timnodonic (eicosapentaenoic) acid (C20:5 n-3; EPA), heneicosapentaenoic acid (C21:5 n-3), clupanodonic acid (C22:5 n-3) and cervonic (docosahexaenoic) acid (C22:6 n-3; DHA). The content of the Fish oil, rich in omega-3-acids is as follows:

EPA, expressed as triglycerides: minimum 13.0 per cent,
DHA, expressed as triglycerides: minimum 9.0 per cent,
Total omega-3-acids, expressed as triglycerides: minimum 28.0 per cent.

Authorized antioxidants in concentrations not exceeding the levels specified by the competent authorities may be added.

Whilst these definitions serve to define particularly preferred compositions of the recited excipients, the skilled addressee will appreciate that the composition of appropriate alternative excipients may also deviate from these exact compositional limits. Excipients of choice should exhibit analogous chemical properties such as the ability to solubilise artemether or arteether at the required concentration, not to degrade the pharmaceutically active ingredients, and to be non-toxic. The excipients should also have analogous physical properties such as at least being liquid at body temperature, and preferably having a suitable viscosity to allow the excipient to be used in preferred spray formulations described below. The viscosity for these applications should be low enough to be capable of atomizing, as described below, when used in a pump spray.

As an example, compositions might consist essentially of artemether or arteether and a pharmaceutically acceptable excipient consisting essentially of a triglyceride, liquid at 37°C, and medium chain triglycerides (as defined herein).

Particularly preferred compositions for use in the invention consist essentially of: artemether or arteether; and one or more pharmaceutically-acceptable excipients selected from the group consisting of: medium chain length triglycerides; short chain triglycerides; and omega-3-marine triglycerides, said composition formulated for transmucosal sublingual, buccal or nasal dosage. The exclusion of significant amounts of other materials (e.g. higher molecular weight lipids) renders a composition that is ideally suited to transmucosal nasal, buccal, and especially sublingual delivery.

More preferred compositions comprise: artemether and a pharmaceutically-acceptable excipient selected from the group consisting of: medium chain length triglycerides; short chain triglycerides; and omega-3-marine triglycerides, said composition formulated for transmucosal sublingual, buccal or nasal dosage, and especially a composition consisting essentially of: artemether and a pharmaceutically-acceptable excipient selected from the group consisting of: medium chain length triglycerides; short chain triglycerides; and omega-3-marine triglycerides, said composition formulated for transmucosal sublingual, buccal or nasal dosage.

These composition are substantially free of water, as the inventors have found, contrary to accepted belief, that water can significantly reduce the shelf-life of the compositions, especially when stored at ambient temperatures. Preferred compositions would have less than 1%(w/w) water, and more preferably less than 0.5%(w/w) water, and most preferably less than 0.1%(w/w) water.

Also in any of these compositions, it is especially preferred that the composition is substantially free of ethanol. Again, the inventors have found that ethanol leads to degradation of the pharmaceutically active components. Preferred compositions in particular have less than 1%(w/w) ethanol, and more preferably less than 0.5%(w/w) ethanol and most preferably less than 0.1%(w/w) ethanol.

Also in any of these compositions, it is preferred that artemether or arteether is present at a concentration of between 2 and 250 milligrams per gram of excipient. This concentration provides an appropriate level for the expected volumes used for the described transmucosal delivery. More preferably, the composition comprises: artemether or arteether, dissolved in the excipient at a concentration of between 2 and 200 milligrams per gram of excipient. Other preferred concentrations are between 2 and 100 milligrams per gram; between 2 and 50 milligrams per gram. The lower concentrations provide compositions particularly suitable for paediatric use, and are also more likely to ensure that the pharmaceutically active components remain in solution over a wide temperature range, rather than having some portion as e.g. a suspension. This is particularly important to ensure that delivery of the drug is by the recited transmucosal route. If significant amounts of the active components are not in solution, then there is an increased likelihood that some will be swallowed, thereby reducing the beneficial effects of such transmucosal delivery described below.

In especially preferred compositions, the said excipient comprises a medium chain triglyceride, said triglyceride comprising a minimum of 95 per cent of saturated fatty acids with between 6 and 12 carbon atoms. More preferably, said excipient comprises a medium chain triglyceride, said triglyceride comprising a minimum of 95 per cent of saturated fatty acids with 8 and 10 carbon atoms.

Also in any such composition, it is also particularly preferred that the composition further comprises an essential oil such as menthol, vanillin or orange oil, lemon oil, clove oil, peppermint oil, spearmint oil. Particular technical advantages of such an essential oil, especially menthol, which acts as a solubilising agent, are described further below.

Also described is a medicament delivery device containing a composition described herein, said device adapted to deliver individual or successive doses of said composition, each individual or successive dose having a volume of less than 1000 microlitres. The use of small dose volumes reduces the likelihood that the composition will be swallowed, or spat out, by the patient. The likelihood is reduced further by use of smaller volumes (especially in the paediatric context or for nasal delivery) and so in further preferred embodiments, each successive dose has a volume of less than 600 microlitres; less than 400 microlitres; less than 200 microlitres; or even less than 100 microlitres. Smaller volumes are especially preferred for paediatric use, or nasal delivery.

Further described is a medicament delivery device containing a composition described herein, said device and composition adapted to deliver individual or successive doses of said composition, each individual or successive dose containing no more than 80mg of artemether or arteether. Such devices are preferably adapted to assist sublingual delivery, especially by non-medically trained personnel. Limiting the amount of active pharmaceutical delivered with each dose is especially important in the context of malaria treatment by less skilled personnel to ensure that over-dosing is avoided. Preferably, said device and composition adapted to deliver individual or successive doses of said composition, each individual or successive dose containing no more than 10mg of artemether or arteether. This provides an appropriate device for paediatric use.

Preferably, the aforementioned delivery devices comprise a spray, and especially a pump spray. The use of a pump spray increases the area of mucosa to which the composition is applied, thereby increasing absorption and minimising the likelihood that the medicament is swallowed. More preferably, said device is adapted to produce a spray of composition having a mean droplet diameter greater than 20 microns, or even greater than 50 microns, or preferably greater than 75 microns. In this way, inadvertent delivery of the medicament to the lungs is avoided, or reduced.

Further described is a device for providing pharmaceutical doses comprising a container containing a pharmaceutical composition described herein, and valve means arranged to transfer doses of said pharmaceutical composition to the exterior of the container. Such a device may be attached to e.g. a separate transmucosal buccal, nasal or sublingual delivery device, such as a spray.

Further described is a kit for the treatment or prophylaxis of malaria comprising a composition described herein and instructions to administer said composition to a patient in need thereof by the transmucosal sublingual, buccal or nasal route. Preferably, said kit has instructions to administer said composition to a patient in need thereof by the sublingual route.

Further described is a method of treating a disease responsive to artemether or arteether (and preferably artemether) comprising the administration to a patient in need thereof of a therapeutically effective amount of artemether or arteether by the transmucosal sublingual, buccal or nasal route. More preferably, said administration is by the sublingual route, and most preferably said disease is malaria.

Further described is a method of treating malaria comprising the administration to a patient in need thereof of a therapeutically effective amount of artemether in the form of a composition as described herein by the transmucosal sublingual, buccal or nasal route, preferably in the form of a spray.

Description and Preferred Embodiments of the Invention

One of the most important aspects of providing a clinically useful treatment for malaria is to provide a formulation and an administration route for any active ingredient that can withstand the challenges of those communities where malaria is an especially acute problem, such as in sub-Saharan Africa. For example, any formulation needs to be stable for long periods of time, and at the relatively high temperatures encountered there. The medicament will often need to be administered (without delay) to children who are weak, malnourished, and likely to be suffering from vomiting and diarrhoea. In many cases, the medicament may also need to be administered by non-medically-trained personnel. It is also important for any active ingredient to have good (and consistent) bioavailability, to ensure that the drug reaches the site of action without adverse side effects.

In order to address these problems, the inventors have found that the transmucosal sublingual, buccal or nasal route of administration of artemether provides a greater likelihood of higher and more reproducible levels of bioavailability than that demonstrated by the oral (i.e. swallowed) or intramuscular route. Navaratnam et al (Clin Pharmacokinet, 2000, Oct; 39(4): 255-270) report the bioavailability of artemether in animals by oral administration to be as low as 19-35%, and only 54% when administered by intramuscular injection. In humans, the bioavailability of artemether was low in both the intramuscular (25%) and intrarectal (35%) route, with considerable variability in absorption. The authors report that *"Preliminary studies in children with cerebral malaria indicated that the bioavailability of intramuscular artemether is highly variable and could potentially affect treatment outcome in the most severely ill patients"*.

The use of the transmucosal sublingual, buccal or nasal route of administration avoids the first-pass effect that occurs with oral and rectal administration. Whilst adults might be able to tolerate the large oral doses of artemether required to overcome the low bioavailability of the drug, this is not the case in children, and so the compositions disclosed herein are particularly suitable for the treatment of malarial in children, i.e. for paediatric formulations.

Preliminary results of initial, confidential, dose ranging studies are presented below, indicating surprisingly increased bioavailability of the drug when administered by sublingual spray in comparison to oral administration by tablet.

The inventors have also found that, contrary to accepted belief, artemether is not stable when in contact with water, ethanol, or propellants that might be used for aerosol formulations.

Tables 1 and 2 show impurities present in Artemether API, and artemether in three solvent systems: 20% ethanol + 80% propellant; 50% ethanol + 50% propellant; 100% ethanol; and a medium chain triglyceride, in this case, the triglyceride sold under the registered trade mark Miglyol® 810. Miglyol® is a medium chain triglyceride containing saturated C8 and C10 fatty acids, typically between 65-80% of caprylic acid (C8:0) and 20-35% of capric acid (C10:0).

The propellant used in these test was 1,1,1,2 tetrafluoroethane, sold under the registered trade mark Zephex® 134a. Similar results were obtained for the propellants butane, Zephex® 227 (1,1,1,2,3,3,3 heptafluoropropane) and for a mixture of butane and propane.

Table 1 shows the impurities (as a percentage of the peak area of an HPLC chromatogram of artemether) after storage of the compositions at 30°C for eight weeks. Table 2 shows the corresponding impurities after storage for eight weeks at 40°C.

*Table 1 - Storage at 30°C*

| Relative Retention Time: | 0.35 | 0.68 | 0.73 | 0.87 | 0.91 | 1.17 |
|---|---|---|---|---|---|---|
| | % of artemether | | | | | |
| Artemether API | 0.4 | | 0.1 | 0.2 | | |
| 20% EtOH 80% propellant | 1.6 | 0.3 | 0.7 | 0.2 | 1.3 | 0.2 |
| 50% EtOH 50% propellant | 1.0 | 0.2 | 0.5 | 0.2 | 1.5 | 0.2 |
| 100% EtOH | 0.3 | 0.2 | 0.5 | 0.2 | | |
| Miglyol 810® | 0.4 | | 0.1 | 0.2 | | |

*Table 2 - Storage at 40°C*

| Relative Retention Time: | 0.35 | 0.68 | 0.73 | 0.87 | 0.91 | 1.17 |
|---|---|---|---|---|---|---|
| | % of artemether | | | | | |
| Artemether API | 0.4 | | 0.1 | 0.2 | | |
| 20% EtOH 80% propellant | 4.9 | 1.9 | 2.9 | 0.2 | 5.3 | 1.4 |
| 50% EtOH 50% propellant | 2.2 | 1.4 | 2.5 | 0.2 | 4.8 | 1.0 |
| 100% EtOH | 2.2 | 0.7 | 1.6 | 0.2 | 1.0 | 0.7 |
| Miglyol 810® | 0.6 | | 0.1 | 0.2 | | |

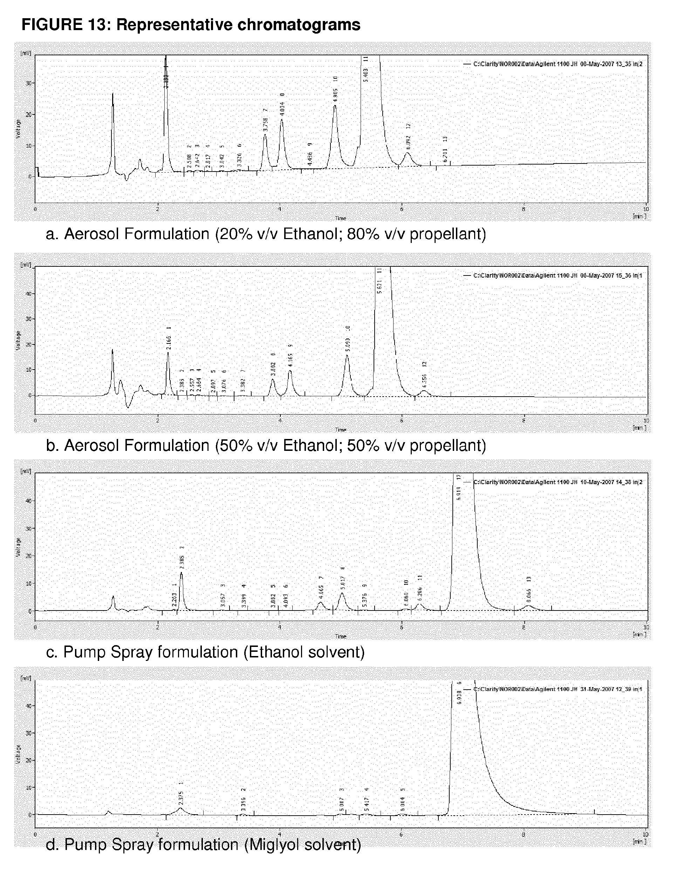

Representative chromatograms are shown in Figure 13. It can be seen that the levels of impurities in the Miglyol® 810 formulation are not significantly higher than those observed in the initial Artemether API. In all other cases, the impurities are at levels that exceed those permitted under the ICH Harmonised Tripartite Guidelines for Impurities in New Drug Products without specific identification or further toxicological examination.

A solution in a medium chain triglyceride, especially a saturated triglyceride such as Miglyol® 810 therefore constitutes a stable formulation for the active ingredient. Being a saturated triglyceride, it is believed that this confers stability to the artemether. Given its chemical structure, it is likely that the main route of degradation of artemether is via reduction mechanisms, which might explain the protection afforded by such saturated fatty acid-containing triglycerides.

When used in a spray delivery system, e.g. in a manually-actuated pump spray, the triglyceride also acts as a pump and valve lubricant, thereby removing the need to add additional lubricants to the formulation. The use of such medium chain triglycerides also produces a formulation of appropriate viscosity and surface tension for use in a pump spray delivery system.

Further advantages also flow from the use of medium chain triglyceride: being hydrophobic, the triglyceride adheres to the mucosa of the mouth, and so allows time for the artemether to be absorbed transmucosally. The hydrophobic nature of the composition resists being washed out of the mouth by the action of saliva, which would otherwise cause the active ingredient to be swallowed.

In especially preferred embodiments of the invention, the artemether-triglyceride solution is supplemented with menthol, or alternatively with orange oil or vanilla. The inventors have found that this has a number of benefits:

(1) Its function as a taste-masking agent is particularly important in the context of administration of anti-malarial drugs to children; the drug is often administered over a number of days, such as a three to five-day regime, and any bad taste of the drug experienced by the child on the first dose, makes it difficult to administer subsequent doses.

(2) The essential oil also acts as a penetration enhancer to improve the uptake of the pharmaceutical ingredient through the mucosa of the mouth.

(3) The addition of a flavour also allows the person administering the drug to check firstly that the drug has been dispensed (the patient can taste or smell it) and secondly that it has been dispensed into the right place - if the drug were e.g. accidentally dispensed directly into the throat, there would be no taste sensation.

(4) A surprising feature is that the essential oil (especially levomenthol) also assists with the solubilisation of the artemether. In a solubility trial, dissolution of artemether in miglyol occurred after 4 minutes 30 seconds when menthol added before artemether compared to 5 minutes 55 seconds when artemether added before menthol.

Preferred formulations (for sublingual or buccal paediatric use) are given in Tables 3 and 4. Two different dose concentrations are given suitable for use in a spray delivery system. A number of sprays (i.e. individual spray actuations of 100microlitres) may be given, dependent on the weight of the child to be treated:

*Table 3: 3mg Artemether per actuation*

| Raw Material Item | Weight (g) | % w/w |
|---|---|---|
| Artemether IP | 0.090 | 3.2 |
| Levomenthol *Ph. Eur.* | 0.020 | 0.7 |
| Miglyol® 810 | 2.690 | 96.1 |

*Table 4: 6mg Artemether per actuation*

| Raw Material Item | Weight (g) | % w/w |
|---|---|---|
| Artemether IP | 0.180 | 6.4 |
| Levomenthol *Ph. Eur.* | 0.020 | 0.7 |
| Miglyol® 810 | 2.600 | 92.9 |

Table 5 outlines an example of a preferred dosage regime for paediatric use. Alternative regimes are envisaged, e.g. dosing at 3mg/kg body weight.

Table 5: Paediatric Dosage Regime

| Weight of child (kg) | Number of doses at 3mg Dose per spray actuation | Total delivered dose mg/kg | Number of doses at 6mg Dose per spray actuation | Total delivered dose mg/kg |
|---|---|---|---|---|
| 3 | 1 | 1.00 | | |
| 4 | 1 | 0.75 | | |
| 5 | 2 | 1.20 | | |
| 6 | 2 | 1.00 | | |
| 7 | 2 | 0.86 | | |
| 8 | 3 | 1.13 | | |
| 9 | 3 | 1.00 | | |
| 10 | 3 | 0.90 | | |
| 11 | 4 | 1.09 | | |
| 12 | 4 | 1.00 | 2 | 1.00 |
| 13 | 4 | 0.92 | 2 | 0.92 |
| 14 | 5 | 1.07 | 2 | 0.86 |
| 15 | 5 | 1.00 | 3 | 1.20 |
| 16 | 5 | 0.94 | 3 | 1.13 |
| 17 | | | 3 | 1.06 |
| 18 | | | 3 | 1.00 |
| 19 | | | 3 | 0.95 |
| 20 | | | 3 | 0.90 |
| 21 | | | 3 | 0.86 |
| 22 | | | 4 | 1.09 |
| 23 | | | 4 | 1.04 |
| 24 | | | 4 | 1.00 |
| 25 | | | 4 | 0.96 |
| 26 | | | 4 | 0.92 |
| 27 | | | 4 | 0.89 |
| 28 | | | 5 | 1.07 |
| 29 | | | 5 | 1.03 |
| 30 | | | 5 | 1.00 |

Formulations for adult use may be prepared at higher concentrations of artemether, such as 150-200 mg/ml. For adult use, individual spray volumes may be larger than the 100microlitre example described here for paediatric use.

Bioavailability of Artemether

The applicant has carried out confidential trials to asses the uptake of the artemether-containing compositions of the present invention when delivered by the sublingual route, by comparison to oral administration by tablet.

Trials were carried out on healthy male adult human volunteers (16 subjects per cohort), and subject to normal ethical approval. Three single-dose regimes according to the present invention were studied, and compared to a regime using oral-dosed tablets, as follows:

Sub-Lingual Spray Regimes

Spray formulations of artemether were prepared as detailed above, and administered, on a single occasion, to a group of volunteers by the sublingual route. A number of successive actuations of the spray were administered, as shown in Table 6, below.

*Table 6 - Dosage Regime for Single Dose Study Sublingual Spray Formulation*

| Test | Formulation | Dose per Actuation (mg) | Number of Actuations | Total Doge (mg) |
|------|-------------|-------------------------|----------------------|-----------------|
| T1 | As Table 3 | 3 | 5 | 15 |
| T2 | As Table 3 | 3 | 10 | 30 |
| T3 | As Table 4 | 6 | 5 | 30 |

Reference Oral Dose

As a reference, a fourth group of volunteers were administered tablets containing artemether, on a single occasion, as shown in Table 7, below.

*Table 7 - Dosage Regime for Single Dose Study Oral Tablet Formulation*

| Test | Formulation | Dose per Tablet (mg) | Number of Tablets | Total Doge (mg) |
|------|-------------|----------------------|-------------------|-----------------|
| T4 | Tablet | 10 | 3 | 30 |

Following administration of each dosage regime, blood samples were taken from the subjects, and plasma concentrations of artemether and its immediate metabolite dihydroartemesinin were determined, in order to compare bioavailability by the two routes.

Figures 1-6 show mean plasma concentration of artemether following two comparison dose regimes. Figures 7-12 show the corresponding mean plasma concentration of dihydroartemesinin.

Figures 1 and 7 compare regimes T1 (open squares) and T4 (closed circles): 15mg artemether via 5 sublingual spray doses vs. 30mg artemether via tablet.

Figures 2 and 8 compare regimes T2 (open squares) and T4 (closed circles): 30mg artemether via 10 sublingual spray doses vs. 30mg artemether via tablet.

Figures 3 and 9 compare regimes T3 (open squares) and T4 (closed circles): 30mg artemether via 5 sublingual spray doses vs. 30mg artemether via tablet.

Figures 4 and 10 compare regimes T1 (open squares) and T2 (closed circles): 15mg artemether via 5 sublingual spray doses vs. 30mg artemether via 10 sublingual spray doses.

Figures 5 and 11 compare regimes T2 (open squares) and T3 (closed circles): 30mg artemether via 10 sublingual spray doses vs. 30mg artemether via 5 sublingual spray doses.

Figures 6 and 12 compare regimes T1 (open squares) and T3 (closed circles): 15mg artemether via 5 sublingual spray doses vs. 30mg artemether via 5 sublingual spray doses).

Pharmacokinetic data for each of the four dosage regimes are given in Tables 8-11, below:

*Table 8: Test Group T1*

| Single sublingual administration of 15mg Artemether sublingual spray: 3mg per actuation | | |
|---|---|---|
| | Plasma Artemether | Plasma Dihydroartemesinin |
| Pharmacokinetic Parameters* | (n=16) (mean $\pm$SD) | (n=16) (mean $\pm$SD) |
| $AUC_{0-12}$ (ng.h/mL) | 25.85 $\pm$ 13.88 | 29.63 $\pm$ 11.58 |
| $C_{max}$ (ng/mL) | 16.11 $\pm$ 8.69 | 18.29 $\pm$ 7.52 |
| $T_{max}$ (h) | 1.70 $\pm$ 0.68 | 1.83 $\pm$ 0.68 |
| $t_{1/2}$ (h) | 0.72 $\pm$ 0.30 | |
| $\lambda_z$ (h$^{-1}$) | 1.11 $\pm$ 0.40 | |
| CL/F (ng/h) | 0.74 $\pm$ 0.46 | 0.54 $\pm$ 0.15 |

(continued)

| Single sublingual administration of 15mg Artemether sublingual spray: 3mg per actuation | | |
|---|---|---|
| | Plasma Artemether | Plasma Dihydroartemesinin |
| Pharmacokinetic Parameters* | (n=16)<br>(mean ±SD) | (n=16)<br>(mean ±SD) |
| V/F (L) | 0.68 ± 0.33 | 0.51 ± 0.16 |
| *Key:<br>$AUC_{0-12}$ (ng.h/mL)    Area under the concentration curve between 0-12 h.<br>$C_{max}$ (ng/mL)    Maximum observed plasma concentration<br>$T_{max}$ (h)    Time of observed maximum plasma concentration<br>$t_{1/2}$ (h)    Elimination half-life<br>$\lambda_z$ ($h^{-1}$)    Elimination rate constant<br>CL/F (ng/h)    Apparent clearance rate<br>V/F (L)    Apparent volume of distribution | | |

*Table 9: Test Group T2*

| Single sublingual administration of 30mg Artemether sublingual spray: 3mg per actuation | | |
|---|---|---|
| | Plasma Artemether | Plasma Dihydroartemesinin |
| Pharmacokinetic Parameters | (n=16) (mean ±SD) | (n=16) (mean ±SD) |
| $AUC_{0-12}$ (ng.h/mL) | 76.60 ± 43.12 | 99.51 ± 50.33 |
| $C_{max}$ (ng/mL) | 32.12 ± 16.39 | 44.11 ± 28.48 |
| $T_{max}$ (h) | 1.73 ± 0.82 | 2.10 ± 1.17 |
| $t_{1/2}$ (h) | 1.39 ± 0.49 | |
| $\lambda_z$ ($h^{-1}$) | 0.56 ± 0.20 | |
| CL/F (ng/h) | 0.56 ± 0.37 | 0.36 ± 0.13 |
| V/F (L) | 1.00 ± 0.55 | 0.72 ± 0.36 |
| Key as Table 8 | | |

*Table 10: Test Group T3*

| Single sublingual administration of 30mg Artemether sublingual spray: 6mg per actuation | | |
|---|---|---|
| | Plasma Artemether | Plasma Dihydroartemesinin |
| Pharmacokinetic Parameters | (n=16) (mean ±SD) | (n=16) (mean ±SD) |
| $AUC_{0-12}$ (ng.h/mL) | 71.11 ± 41.08 | 86.19 ± 27.68 |
| $C_{max}$ (ng/mL) | 35.24 ± 23.91 | 41.14 ± 16.45 |
| $T_{max}$ (h) | 1.67 ± 0.77 | 1.88 ± 0.74 |
| $t_{1/2}$ (h) | 1.40 ± 0.59 | |
| $\lambda_z$ ($h^{-1}$) | 0.59 ± 0.25 | |
| CL/F (ng/h) | 0.63 ± 0.49 | 0.39 ± 0.15 |
| V/F (L) | 1.01 ± 0.49 | 0.91 ± 0.67 |
| Key as Table 8 | | |

*Table 11: Test Group T4*

| Single oral administration of 30mg Artemether Tablets 10mg per Tablet | | |
|---|---|---|
| | Plasma Artemether | Plasma Dihydroartemesinin |
| Pharmacokinetic Parameters | (n=16) (mean $\pm$SD) | (n=16) (mean $\pm$SD) |
| $AUC_{0-12}$ (ng.h/mL) | 34.59 $\pm$ 21.01 | 38.49 $\pm$ 12.38 |
| $C_{max}$ (ng/mL) | 10.12 $\pm$ 7.19 | 10.99 $\pm$ 4.39 |
| $T_{max}$ (h) | 1.02 $\pm$ 0.86 | 1.39 $\pm$ 0.88 |
| $t_{1/2}$ (h) | 3.44 $\pm$ 4.26 | |
| $\lambda_z$ ($h^{-1}$) | 0.31 $\pm$ 0.15 | |
| CL/F (ng/h) | 1.11 $\pm$ 1.01 | 0.76 $\pm$ 0.23 |
| V/F (L) | 3.90 $\pm$ 2.90 | 2.36 $\pm$ 1.26 |
| Key as Table 8 | | |

From these preliminary results, it can be seen that comparison of the area under the plasma concentration curve during the 12 hours following the doses ($AUC_{0-12}$), a well-accepted measure of absorption, shows significant and surprisingly higher absorption of artemether when administered sublingually as a spray formulation as disclosed herein by comparison to oral tablet dosing.

For comparison of bioavailability of artemether via the sublingual spray route described herein with administration by oral tablets, we have calculated the F-values, commonly used to compare two dose regimes, generally A and B, for the artemether data, as follows:

$$F_{A-B} = \frac{AUC_A}{AUC_B} \frac{dose_B}{dose_A}$$

The results are as follows:

$$F_{T1\text{-}T4} = 1.67 \pm 0.60 \text{ (S.D.)}$$

$$F_{T2\text{-}T4} = 2.24 \pm 0.92 \text{ (S.D.)}$$

$$F_{T3\text{-}T4} = 2.09 \pm 0.69 \text{ (S.D.)}$$

This indicates that approximately between 1.7 and 2.2 times more artemether was absorbed when administered as a sublingual spray as described herein by comparison to oral administration by tablet, despite the oral dose being twice as large in the first instance. The indicative bioavailability by the sublingual route is therefore at least twice that by the oral route for equivalent doses.

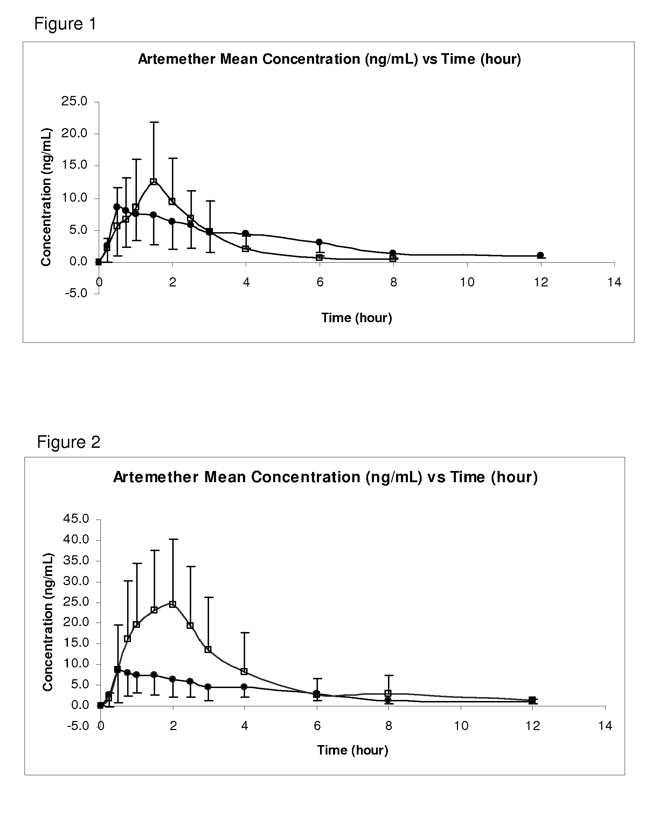

Inspection of the data of Tables 8-11, and Figures 1-12 also confirms this general finding for the primary active metabolite of artemether (dihydroartemesinin).

Avoidance of Autoinduction

It is known that both oral and rectal administration of artemesinins is associated with autoinduction of the drug metabolism in individuals (see e.g. Ashton M, Hai TN, Sy ND, Huong DX, Van Huong N, Nieu NT, Cong LD. "Artemisinin pharmacokinetics is time-dependent during repeated oral administration in healthy male adults.", Drug Metab Dispos. 1998; 26:25-7, and "Retrospective analysis of artemisinin pharmacokinetics: application of a semiphysiological autoinduction model", Asimus and Gordi, Br. J Clin Pharmacol. 2007 June; 63(6): 758-762). As a result, systemically circulating artemesinin declines with each successive dose, thereby reducing the effectiveness of drug dosage regimes.

In confidential trials, the inventors have found that administration of artemesinins by the transmucosal sublingual route avoids such autoinduction, leading to consistent uptake and accumulating systemic concentration of the active drug metabolite, dihydroartemesinin, thereby providing significant advantage in administration by the sublingual route. A similar avoidance of autoinduction is expected with delivery by the transmucosal buccal or nasal route.

In confidential trials, volunteers followed the following treatment: A single administration of 30mg artemether sublingual spray 6mg/actuation on days 1 and 5 following an overnight fast, and twice daily administrations of 30mg artemether sublingual spray 3mg/actuation on days 2, 3,and 4 following a morning or evening meal. Blood samples were collected for pharmacokinetic analysis at the following time points:

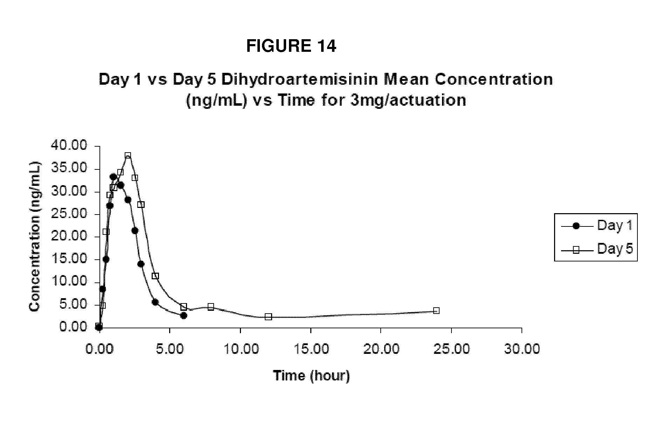

Day 1: Predose, 0.25, 0.5, 0.75, 1, 1.5, 2, 2.5, 3, 4, 6, 8, and 12 h after dosing.
Days 2, 3, and 4: pre morning dose and 0.5, 1, 2 and 4 h after morning dose and pre evening dose and 1 hour after evening dose.
Day 5: Predose, 0.25, 0.5, 0.75, 1, 1.5, 2, 2.5, 3, 4, 6, 8, 12 h and 24 h after dosing. Pharmacokinetic analysis of plasma dihydroartemesinin on days 1 and 5 revealed an effectively identical response, indicating the lack of autoinduction. Plasma concentration curves are shown in Figure 14.

Figure Captions

Figure 1: Plot of mean plasma Artemether concentration vs time with standard deviation following a single sublingual administration of 15mg Artemether Sublingual Spray 3mg/actuation (T1) and single oral administration of 30mg Artemether Tablets 10 mg/tablet (T4). Mean $\pm$ SD (• = reference, T4 , D = test, T1)

Figure 2: Plot of mean plasma Artemether concentration vs time with standard deviation following a single sublingual administration of 30mg Artemether Sublingual Spray 3mg/actuation (T2) and single oral administration of 30mg Artemether Tablets 10 mg/tablet (T4). Mean $\pm$ SD (• = reference, T4 , D = test, T2)

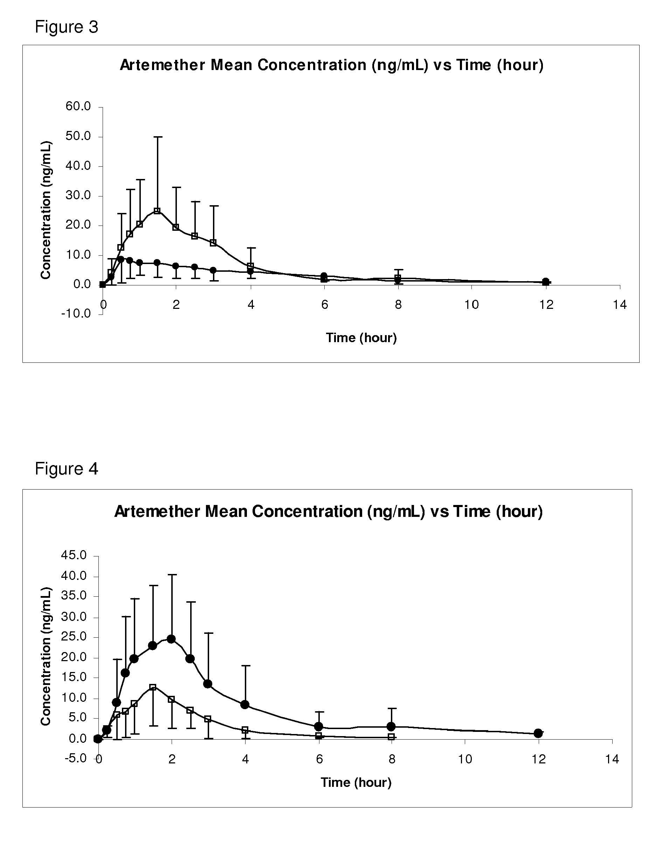

Figure 3: Plot of mean plasma Artemether concentration vs time with standard deviation following a single sublingual administration of 30mg Artemether Sublingual Spray 6mg/actuation (T3) versus single oral administration of 30mg Artemether Tablets 10 mg/tablet (T4). Mean $\pm$ SD (• = reference, T4 , D = test, T3)

Figure 4: Plot of mean plasma artemether concentration vs time with standard deviation following a single sublingual administration of 15mg Artemether Sublingual Spray 3mg/actuation (T1) versus single sublingual administration of 30mg Artemether Sublingual Spray 3mg/actuation (T2). Mean $\pm$ SD (• = reference, T2 , D = test, T1)

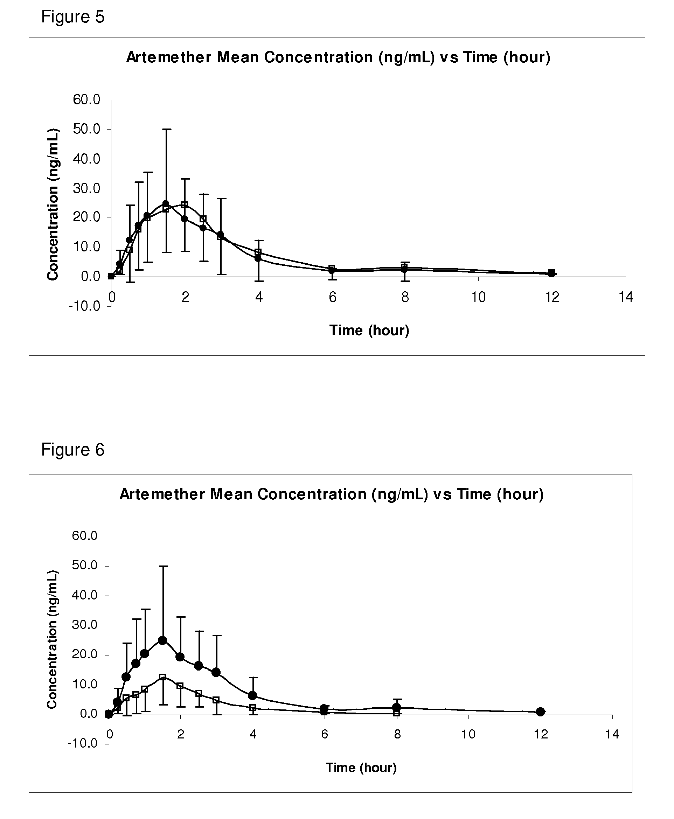

Figure 5: Plot of mean plasma Artemether concentration vs time with standard deviation following a single sublingual administration of 30mg Artemether Sublingual Spray 3mg/actuation (T2) versus single sublingual administration of 30mg Artemether Sublingual Spray 6mg/actuation (T3). Mean $\pm$ SD (• = reference, T3 , D = test, T2)

Figure 6: Plot of mean plasma Artemether concentration vs time with standard deviation following a single sublingual administration of 15mg Artemether Sublingual Spray 3mg/actuation (T1) versus single sublingual administration of 30mg Artemether Sublingual Spray 6mg/actuation (T3). Mean $\pm$ SD (• = reference, T3 , D = test, T1)

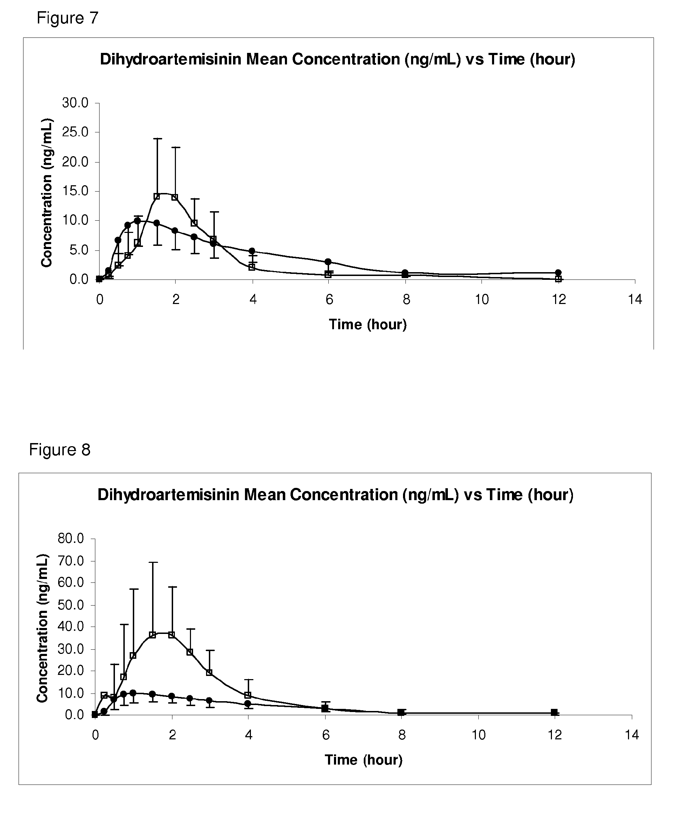

Figure 7: Plot of mean plasma Dihydroartemisinin concentration vs time with standard deviation following a single sublingual administration of 15mg Artemether Sublingual Spray 3mg/actuation (T1) and single oral administration of 30mg Artemether Tablets 10 mg/tablet (T4). Mean $\pm$ SD (• = reference, T4 , D = test, T1)

Figure 8: Plot of mean plasma Dihydroartemisinin concentration vs time with standard deviation following a single sublingual administration of 30mg Artemether Sublingual Spray 3mg/actuation (T2) and single oral administration of 30mg Artemether Tablets 10 mg/tablet (T4). Mean $\pm$ SD (• = reference, T4 , D = test, T2)

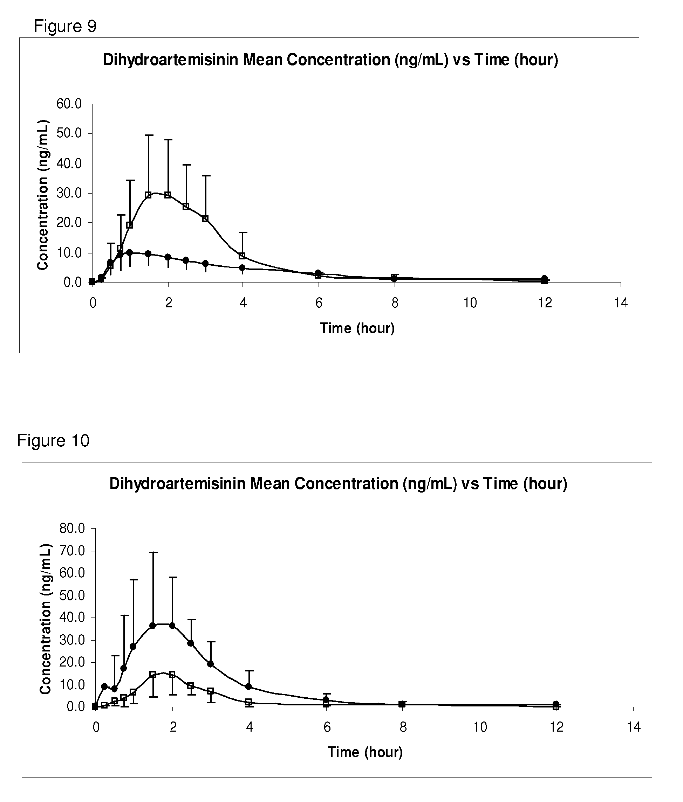

Figure 9: Plot of mean plasma Dihydroartemisinin concentration vs time with standard deviation following a single' sublingual administration of 30mg Artemether Sublingual Spray 6mg/actuation (T3) versus single oral administration of 30mg Artemether Tablets 10 mg/tablet (T4). Mean $\pm$ SD (• = reference, T4 , D = test, T3)

Figure 10: Plot of mean plasma Dihydroartemisinin concentration vs time with standard deviation following a single sublingual administration of 15mg Artemether Sublingual Spray 3mg/actuation (T1) versus single sublingual administration of 30mg Artemether Sublingual Spray 3mg/actuation (T2). Mean ± SD (• = reference, T2 , D = test, T1)

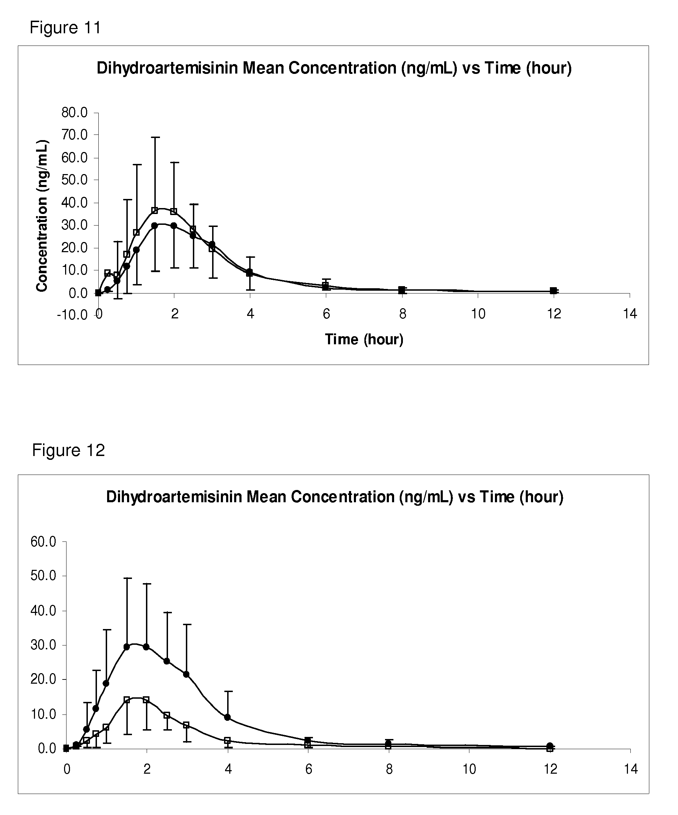

Figure 11: Plot of mean plasma Dihydroartemisinin concentration vs time with standard deviation following a single sublingual administration of 30mg Artemether Sublingual Spray 3mg/actuation (T2) versus single sublingual administration of 30mg Artemether Sublingual Spray 6mg/actuation (T3). Mean ± SD (• = reference, T3 , D = test, T2)

Figure 12: Plot of mean plasma Dihydroartemisinin concentration vs time with standard deviation following a single sublingual administration of 15mg Artemether Sublingual Spray 3mg/actuation (T1) versus single sublingual administration of 30mg Artemether Sublingual Spray 6mg/actuation (T3). Mean ± SD (• = reference, T3 , D = test, T1)

**Claims**

1. A pharmaceutical composition for use in a method of preventing or treating malaria, said composition comprising:

   artemether or arteether; and
   a pharmaceutically-acceptable excipient selected from the group consisting of:

      medium chain length triglycerides;
      short chain triglycerides;
      omega-3-marine triglycerides; and
      fish oil, rich in omega-3-acids

   said composition formulated for transmucosal sublingual, buccal or nasal dosage;
   wherein said composition is substantially free of water.

2. A composition for use according to claim 1 wherein said composition consists essentially of:

   artemether or arteether; and
   one or more pharmaceutically-acceptable excipients selected from the group consisting of:

      medium chain length triglycerides;
      short chain triglycerides;
      omega-3-marine triglycerides; and
      fish oil, rich in omega-3-acids

   said composition formulated for transmucosal sublingual, buccal or nasal dosage;
   wherein said composition is substantially free of water.

3. A composition for use according to claim 1 wherein said composition consists essentially of:

   artemether or arteether; and
   a pharmaceutically acceptable excipient consisting essentially of:

      a triglyceride, liquid at 37°C; and
      medium chain length triglycerides;

   said composition formulated for transmucosal sublingual, buccal or nasal dosage; wherein said composition is substantially free of water.

4. A composition for use according to any preceding claim wherein said composition is substantially free of ethanol.

5. A composition for use according to any preceding claim where in said composition artemether or arteether is present at a concentration of between 2 and 250 milligrams per gram of excipient.

6. A composition for use according to any preceding claim where in said composition said excipient comprises a medium chain triglyceride, said triglyceride comprising a minimum of 95 per cent of saturated fatty acids with between 6 and 12 carbon atoms.

7. A composition for use according to claim 6 wherein said excipient comprises a medium chain triglyceride, said triglyceride comprising a minimum of 95 per cent of saturated fatty acids with 8 and 10 carbon atoms.

8. A composition for use according to any preceding claim wherein said composition further comprises an essential oil such as menthol, vanillin or orange oil, lemon oil, clove oil, peppermint oil, spearmint oil.

9. A composition for use according to any preceding claim wherein said composition is formulated for sublingual delivery.

**Patentansprüche**

1. Pharmazeutische Zusammensetzung zur Verwendung in einem Verfahren zur Vorbeugung oder Behandlung von Malaria, wobei die Zusammensetzung umfasst:

   Artemether oder Arteether; und
   einen pharmazeutisch akzeptablen Hilfsstoff ausgewählt aus der Gruppe bestehend aus:

   Triglyceriden mit mittlerer Kettenlänge;
   kurzkettigen Triglyceriden;
   marinen Omega-3-Triglyceriden; und
   Fischöl, das reich an Omega-3-Säuren ist;

   wobei die Zusammensetzung zur sublingualen, bukkalen oder nasalen Transmukosal-Dosierung formuliert ist;
   wobei die Zusammensetzung im Wesentlichen frei von Wasser ist.

2. Zusammensetzung zur Verwendung nach Anspruch 1, wobei die Zusammensetzung im Wesentlichen aus:

   Artemether oder Arteether; und
   einem oder mehreren pharmazeutisch akzeptablen Hilfsstoffen ausgewählt aus der Gruppe bestehend aus:

   Triglyceriden mit mittlerer Kettenlänge;
   kurzkettigen Triglyceriden;
   marinen Omega-3-Triglyceriden; und
   Fischöl, das reich an Omega-3-Säuren ist, besteht,

   wobei die Zusammensetzung zur sublingualen, bukkalen oder nasalen Transmukosal-Dosierung formuliert ist.

3. Zusammensetzung zur Verwendung nach Anspruch 1, wobei die Zusammensetzung im Wesentlichen aus:

   Artemether oder Arteether; und
   einem pharmazeutisch akzeptablen Hilfsstoff bestehend im Wesentlichen aus:

   einem Triglycerid, das bei 37 °C flüssig ist; und
   Triglyceriden mit mittlerer Kettenlänge;

   besteht,
   wobei die Zusammensetzung zur sublingualen, bukkalen oder nasalen Transmukosal-Dosierung formuliert ist,

4. Zusammensetzung zur Verwendung nach einem der vorhergehenden Ansprüche, wobei die Zusammensetzung im Wesentlichen frei von Ethanol ist.

5. Zusammensetzung zur Verwendung nach einem der vorhergehenden Ansprüche, wobei in der Zusammensetzung Artemether oder Arteether in einer Konzentration von zwischen 2 und 250 Milligramm pro Gramm des Hilfsstoffs vorhanden ist.

6. Zusammensetzung zur Verwendung nach einem der vorhergehenden Ansprüche, wobei in der Zusammensetzung der Hilfsstoff ein Triglycerid mit mittlerer Kettenlänge umfasst, wobei das Triglycerid ein Minimum von 95 % an gesättigten Fettsäuren mit zwischen 6 und 12 Kohlenstoffatomen umfasst.

7. Zusammensetzung zur Verwendung nach Anspruch 6, wobei der Hilfsstoff ein Triglycerid mit mittlerer Kettenlänge umfasst, wobei das Triglycerid ein Minimum von 95 % an gesättigten Fettsäuren mit 8 und 10 Kohlenstoffatomen umfasst.

8. Zusammensetzung zur Verwendung nach einem der vorhergehenden Ansprüche, wobei die Zusammensetzung des Weiteren ein ätherisches Öl wie etwa Menthol, Vanillin oder Orangenöl, Zitronenöl, Pfefferminzöl oder Krauseminzöl umfasst.

9. Zusammensetzung zur Verwendung nach einem der vorhergehenden Ansprüche, wobei die Zusammensetzung zur sublingualen Verabreichung formuliert ist.

**Revendications**

1. Composition pharmaceutique pour une utilisation dans un procédé de prévention ou de traitement du paludisme, ladite composition comprenant :

   de l'artéméther ou de l'artééther ; et
   un excipient pharmaceutiquement acceptable choisi dans le groupe constitué :

      de triglycérides à longueur de chaîne moyenne ;
      de triglycérides à chaîne courte ;
      de triglycérides oméga-3 marins ; et
      d'huile de poisson, riche en acides oméga-3

   ladite composition étant formulée pour une dose administrée par voie transmuqueuse sublinguale, buccale ou nasale;
   dans laquelle ladite composition est essentiellement exempte d'eau.

2. Composition pour une utilisation selon la revendication 1, dans laquelle ladite composition est constituée essentiellement :

   d'artéméther ou d'artééther ; et
   d'un ou de plusieurs excipient(s) pharmaceutiquement acceptable(s) choisi(s) dans le groupe constitué :

      de triglycérides à longueur de chaîne moyenne ;
      de triglycérides à chaîne courte ;
      de triglycérides oméga-3-marins ; et
      d'huile de poisson, riche en acides oméga-3

   ladite composition étant formulée pour une dose administrée par voie transmuqueuse sublinguale, buccale ou nasale.

3. Composition pour une utilisation selon la revendication 1, dans laquelle ladite composition est constituée essentiellement :

   d'artéméther ou d'artééther ; et
   d'un excipient pharmaceutiquement acceptable constitué essentiellement :

      d'un triglycéride, liquide à 37°C ; et
      de triglycérides à longueur de chaîne moyenne ;

   ladite composition étant formulée pour une dose administrée par voie transmuqueuse sublinguale, buccale ou nasale.

4. Composition pour une utilisation selon l'une des revendications précédentes, dans laquelle ladite composition est essentiellement exempte d'éthanol.

**5.** Composition pour une utilisation selon l'une des revendications précédentes où, dans ladite composition, de l'artéméther ou de l'artééther est présent à une concentration comprise entre 2 et 250 milligrammes par gramme d'excipient.

**6.** Composition pour une utilisation selon l'une des revendications précédentes où, dans ladite composition, ledit excipient comprend un triglycéride à chaîne moyenne, ledit triglycéride comprenant un minimum de 95 pour cent d'acides gras saturés avec un nombre d'atomes de carbone compris entre 6 et 12.

**7.** Composition pour une utilisation selon la revendication 6, dans laquelle ledit excipient comprend un triglycéride à chaîne moyenne, ledit triglycéride comprenant un minimum de 95 pour cent d'acides gras saturés avec 8 et 10 atomes de carbone.

**8.** Composition pour une utilisation selon l'une des revendications précédentes, dans laquelle ladite composition comprend en outre une huile essentielle comme le menthol, la vanilline ou l'huile d'orange, l'huile de citron, l'huile de girofle, l'huile de menthe poivrée, l'huile de menthe verte.

**9.** Composition pour une utilisation selon l'une des revendications précédentes, dans laquelle ladite composition est formulée pour une administration par voie sublinguale.

Figure 1

**Artemether Mean Concentration (ng/mL) vs Time (hour)**

Figure 2

**Artemether Mean Concentration (ng/mL) vs Time (hour)**

Figure 3

Artemether Mean Concentration (ng/mL) vs Time (hour)

Figure 4

Artemether Mean Concentration (ng/mL) vs Time (hour)

Figure 5

Artemether Mean Concentration (ng/mL) vs Time (hour)

Figure 6

Artemether Mean Concentration (ng/mL) vs Time (hour)

Figure 7

Dihydroartemisinin Mean Concentration (ng/mL) vs Time (hour)

Figure 8

Dihydroartemisinin Mean Concentration (ng/mL) vs Time (hour)

Figure 9

**Dihydroartemisinin Mean Concentration (ng/mL) vs Time (hour)**

Figure 10

**Dihydroartemisinin Mean Concentration (ng/mL) vs Time (hour)**

Figure 11

**Dihydroartemisinin Mean Concentration (ng/mL) vs Time (hour)**

Figure 12

**Dihydroartemisinin Mean Concentration (ng/mL) vs Time (hour)**

## FIGURE 13: Representative chromatograms

a. Aerosol Formulation (20% v/v Ethanol; 80% v/v propellant)

b. Aerosol Formulation (50% v/v Ethanol; 50% v/v propellant)

c. Pump Spray formulation (Ethanol solvent)

d. Pump Spray formulation (Miglyol solvent)

FIGURE 14

Day 1 vs Day 5 Dihydroartemisinin Mean Concentration
(ng/mL) vs Time for 3mg/actuation

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6306896 B

**Non-patent literature cited in the description**

- **WOODROW et al.** *Postgrad. Med.J.,* 2005, vol. 81, 71-78
- **NAVARATNAM et al.** *Clin Pharmacokinet,* October 2000, vol. 39 (4), 255-270
- **ASHTON M ; HAI TN ; SY ND ; HUONG DX ; VAN HUONG N ; NIEU NT ; CONG LD.** Artemisinin pharmacokinetics is time-dependent during repeated oral administration in healthy male adults. *Drug Metab Dispos.,* 1998, vol. 26, 25-7
- **ASIMUS ; GORDI.** Retrospective analysis of artemisinin pharmacokinetics: application of a semiphysiological autoinduction model. *Br. J Clin Pharmacol.,* June 2007, vol. 63 (6), 758-762